# EUROPEAN PATENT APPLICATION

(11) **EP 2 505 638 A1**
(43) Date of publication of application: **03.10.2012**
(21) Application number: 11002544.2
(22) Date of filing: 28.03.2011
(51) Int. Cl.: C12N 5/00

(54) **Neuronal switching circuit**

(71) Applicant: Technische Universität Berlin, 10623 Berlin (DE)
(72) Inventor: Marx, Uwe, 15528 Spreenhagen (Markgrafpieske) (DE); Lorenz, Alexandra, 13055 Berlin (DE); Hoffmann, Silke, 13187 Berlin (DE)
(74) Representative: Zwicker, Jörk

(57) **Abstract**

The present invention relates to a method of assembling a biological switching circuit (1). In a preferred embodiment the invention comprises assembly of a tissue capable of producing a signal, an afferent signal transmitter, neurons which can act as a control centre (4), an efferent signal transmitter and a tissue which is able to respond to the neuronal signal. The invention also relates to a biological switching circuit (1) producible by the method of the invention and its use.

## Description

### Introduction

The present invention relates to a method of assembling a biological switching circuit (1). In a preferred embodiment the invention comprises assembly of a tissue capable of producing a signal, an afferent signal transmitter, neurons which can act as a control centre (4), an efferent signal transmitter and a tissue which is able to respond to the neuronal signal. The invention also relates to a biological switching circuit (1) producible by the method of the invention and its use.

### Background of the Invention

The organ regulation in the human organism takes place via complex neuronal switching circuits. The easiest configuration is composed of a signal-sending organ, a conglomerate of receiving and assimilating neurons and a response back into the organ or into another organ. During evolution nature developed for this easy circuit and all overlying higher complex neuronal circuits' highly efficient biological building blocks *in vivo* represented by ganglia of the enteric, symphatic and parasympathic nervous systems and the neuronal structures of spinal cord and brain. For almost hundred years mankind has tried to emulate neuronal interactions by tissue culture of networks of neurons (Harrison RG. 1907. Experiments in transplanting limbs and their bearings on the problems of the development of nerves. J Exp Zool 4:239-281). The state of the art of *in vitro* reconstitution/characterization of the nervous system is characterized by three different types of tissue culture methods: a) culturing of brain slices, b) culturing of monolayer culture of neurons with and without glia cells and c) three-dimensional neuronal tissue culture, which in the following will be discussed in more detail. Organotypic brain-slice cultures have been used as models to investigate mechanisms and treatment strategies for neuronal disorders (Gähwiler, Trends in Neuroscience (1997) 20,471-477 and Stoppini et al., Journal of Neuroscience Methods, 37 (1991) 173-182). At the beginning of this century it has been described that slice cultures display more in vivo-like mechanisms of excitotoxicity than reported for primary neuronal cultures (Kristensen et al., Brain Research 917 (2001) 21-44).

This type of culture seems particularly well suited for developmental studies of synaptic connectivity and plasticity of neurons. However, tissue slicing leads to an irreversible cut of individual axons and corresponding nerve fibres. These can not be regenerated *in vitro* and, therefore, this technology is limited to the study of interaction between individual intact neurons in the remaining healthy tissue.

In contrast the approach of co-culturing neurons and glia cells in monolayers as described by Jane Faria et al. (Differentiation (2006) 74:562-572) aims to build neuronal interactions *de novo in vitro*. This technology has led to two-dimensional networks of neurons in tissue culture plates supported by glial connective tissue. These networks, however, only provide data on individual neuronal interactions, which can neither react to a physiological stimulus nor act on a physiological response tissue.

To overcome the limitations of the non-physiological two-dimensional network in monolayer-cultures significant efforts have been devoted over the last decade to provide neurons with an environment and/or scaffold allowing them to arrange in a three-dimensional space. Progress in that respect has been made on tissue formation as described by Preynat-Seauve et al. (Stem Cells 2009; 27:509-520). Preynat-Seauve *et al*. use air-liquid interface culture to engineer three-dimensional human nervous tissues from human embryonic stem cells. An important feature of this technique is the improved exchange between air and tissue. This allows the development of a relatively thick three-dimensional culture and yields dense interconnected neural tissue with structurally and functionally distinct areas. But even this three-dimensional arrangement of neurons in an *in vitro* developed organotypic tissue culture represents only a poor model of the neuronal control centre in man - the ganglion.

None of these technologies has been able to emulate a neuronal switching circuit consisting of a somatic tissue sending a signal through nerve fibres into a neuronal control centre and a somatic tissue responding to a corresponding signal of the neuronal control centre.

Surprisingly a biological switching circuit (1) consisting of a biological sender (3), an afferent transmission line (6), a control centre (4), an efferent transmission line (7) and a responder tissue can be established outside the organism according to the method of this invention. A biological switching unit comprising this biological switching circuit (1) can be used to study neuronal function and also provides a test system for performing any kind of safety and efficacy substance evaluation including lead selection for new pharmaceuticals. Furthermore the biological switching circuit (1) as a whole or parts thereof can be implanted in organisms, preferably immunological compatible organisms, for complementation, restoration and/or regeneration of neuronal functions. Moreover fully functional transplants can be produced on the basis of one or more such biological switching units. The biological switching unit furthermore can be used to complement and/or to substitute technical systems comprising technical switching circuits.

### Brief Summary of the Invention

In a first aspect the present invention relates to a method for assembly of an *in vitro* biological switching circuit (1), comprising the step of seeding a control centre (4), comprising one or more cells capable of forming an afferent transmission line (6), one or more cells capable of forming an efferent transmission line (7) and one or more connective tissue cells (13), wherein the afferent transmission line (6) formed is capable of connecting at least one sender (3) to the control centre (4) in a way allowing transmission of a signal to the control centre (4) and the efferent transmission line (7) formed is capable of connecting at least one responder (5) to the control centre (4) in a way allowing transmission of a signal to said at least one responder (5).

In a second aspect the present invention relates to an *in vitro* biological switching circuit (1) producible by the described method.

In a third aspect the present invention relates to a kit comprising the biological switching unit comprising the biological switching circuit (1) further comprising devices for monitoring (21) the activity of the control centre (4), the sender (3) and/or the responder (5).

In a fourth aspect the present invention relates to the use of the biological switching circuit for research and drug development on neuronal diseases, for safety and efficacy testing of substances, as an implant, as a transplant and as a substitute of technical systems comprising technical switching circuits.

### Detailed Description of the Invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001 and Jörg Knäblein (Modem Biopharmaceuticals - Design, Development and Optimization, 1. Edition, August 2005 Wiley-VCH, Weinheim).

Throughout this specification and the claims which follow, unless the context requires otherwise, the word "comprise", and variations such as "comprises" and "comprising", will be understood to imply the inclusion of a stated feature, integer or step or group of features, integers or steps but not the exclusion of any other feature, integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

In the following, some definitions of terms frequently used in this specification are provided. These terms will, in each instance of its use, in the remainder of the specification have the respectively defined meaning and preferred meanings:
"Afferent'' means carrying toward.
"efferent neurons" which are also referred to as sensory or receptor neurons are neuronal cells that carry a nerve impulse, preferably from various sensory cells, towards the central nervous system or the enteric nervous system. Preferably afferent neurons connect to efferent neurons, for input transmission to a responder tissue or they connect to interneurons, which are also referred to as relay neurons or association neurons. In this case the interneuron is able to mediate nerve impulses of a second switching circuit into the described biological switching circuit.
"Afferent transmission line" comprises one or more afferent neurons and optionally one or more glia cells, which preferably coat or surround the one or more efferent neurons.
"Axon" is the single outgrowth of a neuronal cell, which is preferably ending within a non-neuronal tissue to receive or send a signal.
"Cells" means cell lines or primary cells of vertebrates or invertebrates.
"Control and/or distribution centre" means cluster of neurons and incoming and leaving nerve fibres.
"Common structure" means cells, preferably enclosed in a membrane, with stabilizing, connective tissue cells and with the ability to be in contact sender and/or responder via cell appendices, e.g. axons.
"Efferent" means carrying away.
"Efferent neurons", which are also referred to as motor or effector neurons are neuronal cells that carry a nerve impulse away from the central nervous system or the enteric nervous system. Preferably efferent neurons connect to an afferent neuron to receive an impulse or to interneurons, which are also referred to as relay neurons or association neurons. In this case the interneuron is able to mediate nerve impulses of a second switching circuit into the described biological switching circuit.
"Efferent transmission line" comprises one or more efferent neurons and optionally one or more glia cells, which preferably coat or surround the one or more efferent neurons.
"Matrix" means substances or mixtures of substances, which provide a spatial architecture, maintain viability and tissue specificity, enhance proliferation, differentiation, and function of cells and/or organoid or organ formation such as collagen, fibrinogen, basal lamina, connective tissues and/or synthetic polymers.
"Medium" (plural form: "media") means growth supporting or viability maintaining liquid with nutrients and substances for cultivation of cells. Examples of suitable media comprise DMEM, Ham's F12, RPMI and plasma of different origin.
"Nerve" is an axon and/or a number of axons coated or non-coated with glial cells.
"Nerve impulse" also referred to as "action potential" is a short-lasting event in which the electrical membrane potential of a nerve cell rapidly rises and falls.
"Organoids" means artificial, de novo generated, functional cell aggregates of different types of cells in vitro that show at least one organ or tissue function, preferably shows the majority of organ or tissue functions.
"Responder" means cells and tissues, which are able to respond to a nerve impulse received from efferent neurons, preferably by contraction/relaxation or secretion.
"Sender" means cells and/or tissues, which are able to stimulate a nerve impulse in afferent neurons, preferably in response to tension, pressure, changes in volume, shear stress, nutrients concentration or change thereof, osmolarity, pH, temperature, CO₂ concentration or change thereof, O₂ concentration or change thereof, biochemical changes, light, odor, acoustic noise, taste, colour, and wavelength.
"Substance" means a matter of definite composition and properties.
"Technical switching circuit" means any non-biological switching circuit (1) containing or comprising one or more of the following: a technical sensor (20), a biological circuit interface for providing and/or receiving electrical action potential from neurons, a signal input, a signal processing unit also termed central processing unit (CPU), a signal output and an actor, such as electrical circuits, computational circuits, and hybrid switching units combining for example physical and chemical signals.
"Tissues" means biopsy material or explants taken from patients or animals or in vitro generated tissues.
"Transmitter" means a carrier of nerve impulses across a synapse to a postsynaptic element, such as smooth and/or skeletal muscle, glands or neuronal tissue.

To overcome limitations associated with prior art methods, the present invention provides a method for assembly of an *in vitro* biological switching circuit (1), comprising the step of seeding a control centre (4), comprising one or more cells capable of forming an afferent transmission line (6), one or more cells capable of forming an efferent transmission line (7) and one or more connective tissue cells (13), wherein the afferent transmission line (6) formed is capable of connecting at least one sender (3) to the control centre (4) in a way allowing transmission of a signal to the control centre (4) and the efferent transmission line (7) formed is capable of connecting at least one responder (5) to the control centre (4) in a way allowing transmission of a signal to said at least one responder (5).

The present invention preferably also comprises the step of providing at least one sender (3) and/or at least one responder (5). The sender (3) is preferably a cell, a group of cells, or a tissue, which is generating a nerve impulse if it stimulated by one or more stimuli of the typical five senses - sight (ophthalmoception), hearing (audioception), taste (gustaoception), smell (olfacoception or olfacception), and touch (tactioception) or any of the additional senses such as temperature (thermoception), kinesthetic sense (proprioception), pain (nociception), balance (equilibrioception) and acceleration (kinesthesioception) connected to the afferent transmission line (6). The respective cell, group of cells or tissues generating nerve impulses in response to a stimulus are preferably tissue explants and/or extracted single cell suspensions of the respective cell types maintain in tissue culture. Table I below provides examples of suitable cell types and the respective sense and stimulus.

**Table I**

| Sense | Stimulus | Cell type | Architecture |
|---|---|---|---|
| sight | light | cells of the retina, such as retinal pigment epithelial cells, photoreceptor cells, bipolar cells | composed in the ten distinct layers of vertebrate retina |
| hearing | acoustic noise | hair cells | cochlea shape |
| taste | biochemical changes, nutrients, osmolarity, pH | cells of the taste bud, such as basal cells, gustatory receptor cells including gustatoring hair, supporting cells | taste bud |
| smell | odor | smell nerve cells | tiny patch of tissue |
| touch | pressure, vibration, changes in volume, shear stress, temperature, | Mechanoreceptors or mechanonociceptors | Golgi tendon organs and neuromuscular spindles in skeletal muscles, Meissner, Pacinian, Merkel and Ruffini corpuscles in the skin |

The responder (5) is a cell, a group of cells or a tissue which is capable of responding to the neuronal signal of the control centre (4), preferably selected from the group consisting of smooth and/or skeletal muscle cells, adenocytes, mucosa cells, neuronal cells, a vascular system, nervous system and/or a lymphatic system.

In a preferred embodiment of the method for assembly of an *in vitro* biological switching circuit (1), the one or more cells capable of forming an afferent signal transmission line comprise, consist or essentially consist of afferent neuronal cells (8), afferent neuronal cells (8) and glia cells (10), axons (11) of afferent neuronal cells (8) coated with glia cells (10), and/or bundles of axons (11) of afferent neuronal cells (8) with or without glia cell (10) coating combined into nerves (12) . Preferably, the afferent transmission lines (6) are afferent nerve fibres. These nerves (12) are capable of forming an afferent transmission line (6) and are capable of connecting at least one sender (3) to at least one control centre (4). To promote the growth of the afferent signal transmission line it is preferred that the neuronal cells are selected from the group of differentiated cells derived from donor and/or prenatal neuronal cells differentiated *in vitro.*

The growth of these nerves (12) towards and/or into a group of sender (3) cells or a sender (3) tissue is preferably stimulated by the addition of nerve growth factor into the group of sender (3) cells or sender (3) tissue located in the vicinity of the cells capable of forming an afferent transmission line (6). Preferably the cells or tissue forming the sender (3) are seeded at a distance towards the cells capable of forming an afferent transmission line (6) of less than 100 mm, preferably less than 30 mm. An alternative approach to promote growth of the cells forming the afferent transmission line (6) towards the sender (3) and to electrically connect the afferent transmission line (6) with sender (3) tissue or cells is to separately pre-incubate a control centre (4) formed according to the method of the invention and to add nerve growth factor in semi-solid medium at the later position of the sender (3) tissue. It is preferred that the sender (3) cell, cells or tissue is added once the afferent transmission line (6) has grown into the sender (3) culture space. In either embodiment it is preferred that both the growth space for the control centre and the growth space for the sender (3) is connected by a channel. Preferably the length of such a channel is between 500 mm and 5 mm, preferably between 300 mm and 10 mm, more preferably between 100 mm and 20 mm. To be able to place all elements of the switching circuit generated according to the method of the present invention on a small device, it is preferred that the length of the channel is less than 30 mm.

In a preferred embodiment of the method for assembly of an *in vitro* biological switching circuit (1), the cells capable of forming an efferent transmission line (7) comprise, consist or essentially consist of efferent neuronal cells (9), efferent neuronal cells (9) and glia cells (10), axons (11) of efferent neuronal cells (9) coated with glia cells (10), and/or bundles of axons (11) of efferent neuronal cells (9) with or without glia cell (10) coating combined into nerves (12). Preferably, the signal transmission lines are efferent nerve fibres. To promote the growth of the efferent signal transmission line it is preferred that the neuronal cells are selected from a group of differentiated cells derived from donor and/or prenatal neuronal cells differentiated *in vitro.* The growth of these cells towards and/or into a group of responder (5) cells or responder (5) tissue is preferably stimulated by the addition of nerve growth factor into the group of responder (5) cells or responder (5) tissue located in the vicinity of the cells capable of forming an afferent transmission line (6). Preferably the cells or tissue forming the responder (5) are seeded at a distance towards the cells capable of forming an efferent transmission line (7) of less than 100 mm, preferably less than 30 mm. Another approach to promote growth of the cells capable of forming an efferent transmission line (7) in order to connect the efferent transmission line (7) to responder (5) tissue or responder (5) cells is to separately pre-incubate a control centre (4) and add nerve growth factor in semi-solid medium at the later position of the responder (5) tissue. In this case the responder (5) tissue is added once the axon (11)/nerve (12) growth has arrived in the responder (5) culture space. In either embodiment it is preferred that both the growth space for the control centre and the growth space for the responder (5) is connected by a channel. Preferably the length of such a channel is between 500 mm and 5 mm, preferably between 300 mm and 10 mm, more preferably between 100 mm and 20 mm. To be able to place all elements of the switching circuit generated according to the method of the present invention on a small device, it is preferred that the length of the channel is less than 30 mm.

In a preferred embodiment of the method for assembly of an *in vitro* biological switching circuit (1), the cells forming the control centre (4) comprise, consist or essentially consist of at least one afferent neuronal cell (8), one efferent neuronal cell (9), and at least one connective tissue (13) cell arranged in a common structure. Preferably, the connective tissue (13) cell is a glia cell (10). Preferably the neuronal cells are derived from differentiated neuronal cells, derived from adult organism or prenatal cells derived from neuronal tissue of developing organisms whereas glia cells (10) preferably derived from prenatal glial tissue.

In a preferred embodiment the cells forming the control centre (4) are seeded simultaneously or subsequently into an area of a growth matrix (14), preferably a macro-porous matrix (14) providing globular and/or open cell culture space of 50 µm to 3 mm diameter, preferably from 100 µm to 250 µm. The preferred shape of the culture space for the control centre is spheric or star-like shape. Preferred materials of such matrices are selected from the group consisting of acellularised human ganglion tissue and biodegradable scaffolds. To support and direct the arrangement of neuronal and connective tissue (13) cells, preferably glia cells (10), within such matrix (14) structures these structures are preferably coated with connective tissue matrix or connective tissue (13) cells. Such an arrangement of cells, matrices and coating allows maintaining the control centre (4) in behaviour comparable to a human ganglion.

In a further preferred embodiment of the method of the present invention the control the cells seeded to form the control centre further comprise at least one interneuron. Preferably, an interneuron is seeded, of two or more interconnected biological switching circuits are to be connected.

To ensure proper arrangement of cells into three-dimensional neuronal networks cells have to be seeded with a density of 1 x 10⁶ to 1 x 10⁹ cells per cm³ effective cell culture space, preferably 1 x 10⁸ to 5 x 10⁸. The ratio between neuronal cells and glia cells (10) is preferably between 2:1 and 5:1, preferably 3:1. These are the preferred ratios for the establishment of a control centre. A cell culture period of between 2 and 10 days is necessary for the proper arrangement of the three cell types, i.e. afferent and efferent neuron(s) and connective tissue cell(s), preferably glia cell(s) within the control centre (4). During the establishment of the control centre (4) it is preferred that no other somatic tissues such as sender (3) or responder (5) cells or tissues are cultivated in their corresponding cell culture cavities or alternatively that the cavities for the control centre (4), the sender (3) and/or the responder (5) are fluidically disconnected during this incubation step. Fluidic disconnection may be achieved by placing valves in the channels connecting the cell culture spaces for the control centre (4), the sender (3) and the responder (5). It is preferred that an exchange of culture media is carried out periodically. The amount and frequency is chosen in such to be sufficient to support cell viability.

As set out above, it is preferred that once the cell arrangement in the control centre (4) culture cavity is completed that then the afferent and efferent transmission lines (7) have to be formed by the outgrowth of axons (11) from the neuronal cells of the control centre (4) and the connection of their endings into the sender (3) and/or responder (5) tissue.

In a preferred embodiment the control centre (4) formed resembles the structure of a human ganglion, preferably a ganglion of the enteric nervous system, more preferably a ganglion of the *plexus myentericus*, and/or a ganglion of the *plexus submucosus.* The enteric nervous system is one of the three divisions of the autonomic nervous system, the other being the sympathetic and parasympathetic. One major advantage in contrast to other divisions is that the enteric nervous system can perform many functions independently of the central nervous system (Furness and Costa, Neuroscience, 5 (1980), 1-20), so it is the preferred system for the assembly of an *in vitro* biological switching circuit (1). Accordingly, in a preferred embodiment the cells used for seeding of the control centre are derived from the enteric nervous system or differentiated into such cells.

In the preferred established ganglion emulating the *plexus myentericus* the responder (5) is/are one or more smooth muscle cells maintained in tissue culture in a physiological arrangement. In another embodiment the control centre (4) may be a ganglion emulating the *plexus submucosus.* In this case the responder (5) is/are one or more gland cells of the mucosa maintained in tissue culture in a physiological arrangement.

Both of these plexuses together have an effect on the blood flow and on the interactions with the immune system of the intestine *in vivo.* Direct communications between *plexus myentericus* and *plexus submucosus* ascertain that secretion, blood perfusion and muscle contraction during the propulsion of the faeces are carried out in a coordinated fashion. In a preferred embodiment a biological switching unit modelling the *plexus myentericus* and a biological switching unit modelling the *plexus submucosus* are seeded in culture spaces adjacent to each other and are preferably seeded togther with one or more interneurons, which will provide an interaction between the two plexuses.

In a preferred embodiment the one or more cells involved in the method for assembly of an *in vitro* biological switching circuit (1) are seeded within a three-dimensional structured matrix (14) and/or on a structured surface. Preferably the matrix (14) and/or the structured surface comprise or consist of matrigel, fibrin gel, agarose gel, alginate gel, synthetic gel and/or crosslinkable polymers.

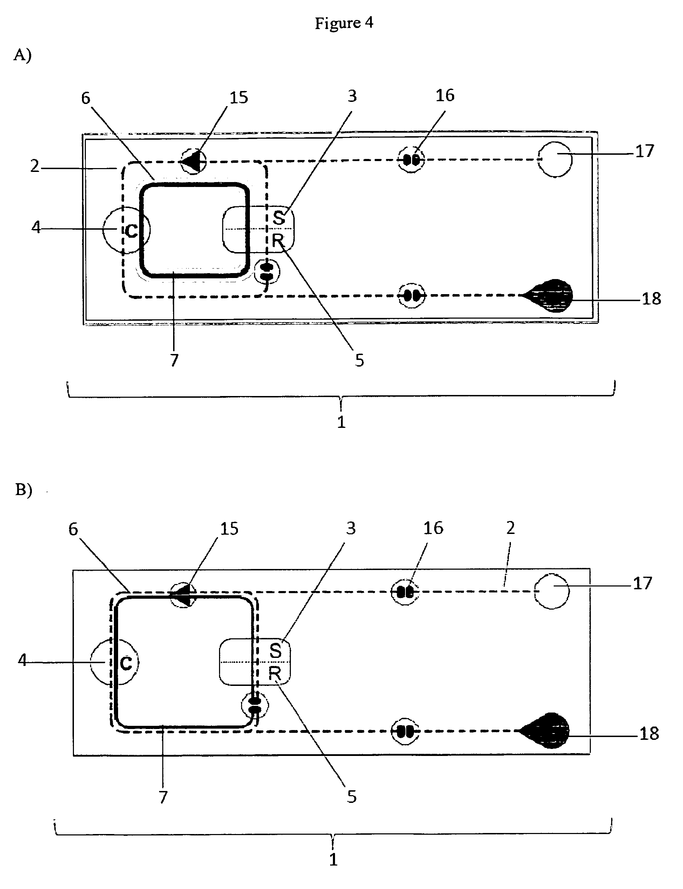

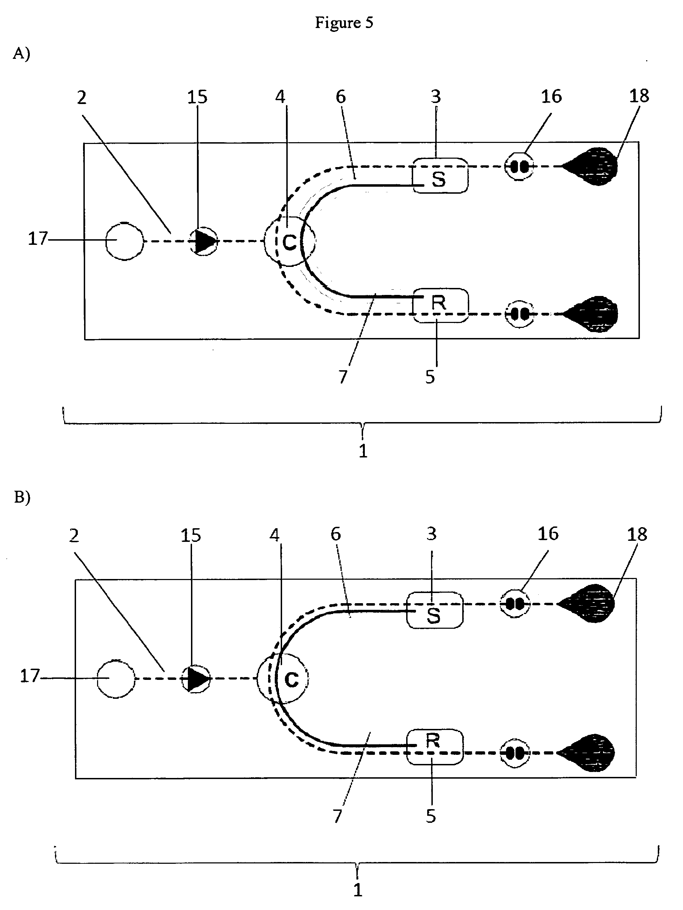

Preferably, the cell cavities for the elements that are assembled into according to the method of the present invention are all comprised in a self-contained device. An example of such a device, i.e. a device which provides structured matrix (14) is depicted in figures 4 and 5. In a preferred embodiment the culture spaces for the control centre (4), the sender (3) and the responder (5) and optionally separate medium flow channels are micro-machined into a suitable material. Preferably, such material is translucent to allow monitoring of the progress of assembly as well as the integrity of the switching circuit (1) once assembled. Suitable materials comprise glass, polystyrol or polycarbonat. As set out above the culture spaces may be filled completely or in part with matrices supporting the growth of the cells to be seeded. If the culture spaces are filled entirely it is preferred that the matrices are biodegradable to allow the ingrowth of the cells.

To provide the cells with oxygen oxygenated medium may be supplied to the cells. This is preferred, if the culture system is closed towards the environment. Alternatively, the culture spaces and/or the channels for transmission line formation may be designed in such to allow gas exchange, i.e. they are either completely or partially open to the environment or they are covered by a membrane that allows gas exchange (O₂/CO₂).

To monitor functionality of such a biological switching circuit (1) methods to measure the signalling of the sender (3), the signal transmission to the control centre (4), the amplification of the signal in the control centre (4) e.g. the neuronal action potential, the transmission of the signal to the responder (5) and the response of the tissue. Preferably the signalling of the sender (3) will be monitored as outlined below:
tension- microscopic observation
pressure - pressure sensor
changes in volume -volume measurement
nutrients - glucose/lactate/amino acid measurements
osmolarity - osmolarimeter
pH - pH-sensor
temperature - temperature sensor, infrared sensor,
CO₂ O₂ - optical measurements,
light -photo/light
odor - measurement of single odor molecules by mass spectrometry
acoustic noise - acoustic sensor
colour, wavelength - wavelength/optical sensors

Signal transmission through afferent and efferent transmission lines (7) and action potential generation in the control centre (4), preferably can be monitored by electroencephalogram, microelectrode arrays (MEA) or optical sensors (20) measuring the excitation of substances sensitive to neuronal action potentials e.g.

Finally the monitoring (21) of the responder (5) preferably can be carried out by smooth and skeletal muscle contraction and relaxation (preferably microscopy, pressure and volume measurements), secretory tissue (quantitative measurement of secretory compounds of glands e.g. ELISA-technology) and neuronal tissue (methods as described for the control centre (4)).

Electrical methods for measuring system parameters are important in diagnosing the status of biological organisms, including humans (Gusev, Biomedical Measurements 8 (1993) 60-62). They assess the electrical resistance of appropriate tissue regions. The value of various parameters and their variation with time yield information's on the state of for instance a special organism, specific organs and systems.

Preferably, the three-dimensional structured matrix (14) and/or structured surface provides an interface for connecting the biological switching unit to at least one technical switching circuit, preferably an electric or computational circuit, i.e. a biological interface. Such an interface allows the input or read-out of electric signals into the biological switching circuit (1). For example, the technical switching unit can receive and process signals received from a biological switching circuit (1) comprising a mechanonociceptor as sender (3) and output information on the tension/pressure thus sensed. A typical technical switching unit used in the context of the present invention comprises a bus system for communication, a data storage device, e.g. hard drive, RAM and/or ROM, and a central processing unit (CPU). Preferably the technical switching unit further comprises an output device, e.g. a graphic processing unit (GPU) and a display unit.

In a preferred embodiment the method for assembly of an *in vitro* biological switching circuit (1) comprises at least one of the steps of co-culturing extracted neuronal cells with glia cells (10) supported by a defined assembly architecture, promoting the signal transmission line growth to the sender (3) and the responder (5) cells/tissues, coupling into the sender (3) and/or responder (5), inducing an impulse of the sender (3) and/or measurement of the response at the receiver (19). To monitor the status of the system it is preferred to cultivate the *in vitro* biological switching circuit (1) on microelectrode arrays (MEA). Morefield et al. (Biosensors & Bioelectronics 15 (2000) 383-396) described that neuronal networks cultured on MEAs remain pharmacologically histiotypic and can provide reliable, quantitative data on drug interactions with neural tissue. They use the system as a test platform for toxicological studies, for drug development and a tissue-based biosensor (20).

The present invention further provides an *in vitro* biological switching circuit (1) producible by the described method for assembly of an *in vitro* biological switching circuit (1). It is one advantage of the invention, that this *in vitro* network has fully human characteristics.

Furthermore, the invention provides a kit comprising the biological switching unit comprising the biological switching circuit (1) and further comprising devices for monitoring (21) the activity of the control centre (4), the sender (3) and/or the responder (5). Such devices may comprise sensor (20) for connecting to the control centre (4), which can sense, e.g. ion fluctuations within the cells forming the control centre (4).

To overcome the limitations associated with prior art, the present invention provides the use of the biological switching circuit (1) and/or unit for research and drug development on neuronal diseases such as Alzheimer, Parkinson, symptoms of neurodegeneration, for safety and efficacy testing of substances, as an implant and a transplant and may enable identification of candidate therapeutic agents.

The present invention further provides an *in vitro* biological switching circuit (1) and/or unit which serves as a physiological model that can be used to characterise the physiology of various organs and/or tissues such as intestine, retina, skin, lung, hair cells of the ear, secretoric glands, blood vessels, muscle, immune tissues, liver, kidney and others.

Alternatively, the present invention of the biological switching circuit (1) and/or unit can further be used as a substitute of technical systems comprising technical switching circuits. One possible application is the substitution of technical circuits comprising temperature sensors in temperature zones of 37°C used for exact measurement of activity of metabolic processes with a respective process control. The use of the biological switching circuit (1) may increase both sensitivity and the dynamic of the control of such systems.

It is to be understood that the above described embodiments are intended to illustrate rather than limit or restrict the scope of the disclosure. Variations may be made to the embodiments without departing from the spirit of the disclosure as claimed.

### Brief Description of the Figures

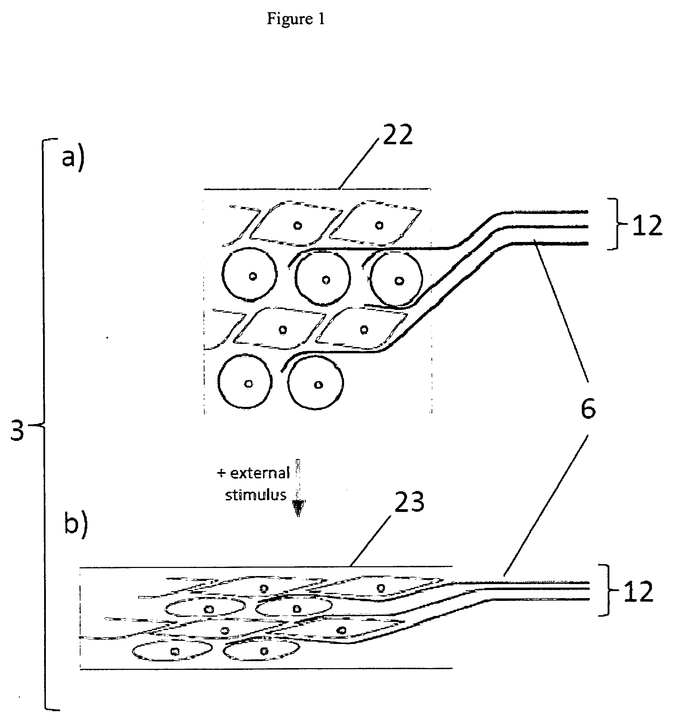

- Fig. 1:: Depicts a schematic drawing of a preferred configuration of a sender (3) tissue. a) Axons of the afferent transmission lines (6) are ending in between of smooth muscle layers of human gut (22). b) The mechanical extension of this in vitro smooth muscle layers leads to nerve impulses (generation of action potentials), signalling through the nerve (12) into the respective afferent neuronal cell bodies located in the control centre.

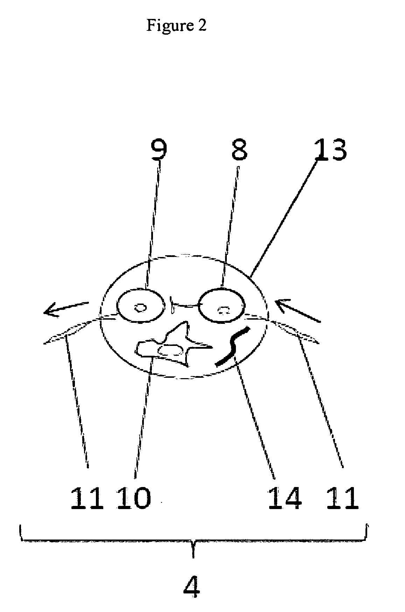

- Fig. 2:: Depicts the control centre (4), which is formed by co-culture of at least one afferent neuron (8), one efferent neuron (9) and one connective tissue cell (glia cell) (10), architecturally arranged in a three-dimensional spheroid matrix (14) housing the cells and allowing exit of growing axons (11). This ganglion-like control centre (4) is preferably coated with matrix proteins or a connective tissue cell layer (14).

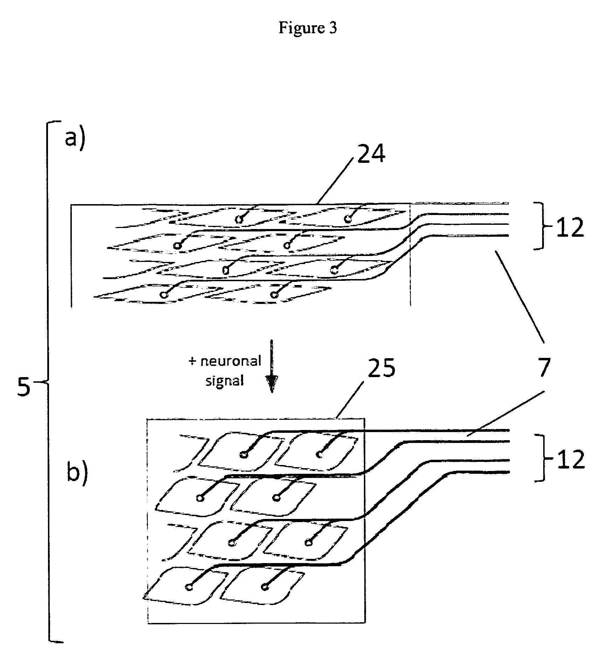

- Fig. 3:: Depicts a schematic drawing of a preferred configuration of a responder (5) tissue. a) Axons of the efferent transmission lines (7) are connected through synapses allowing release of transmitters to smooth muscle cells of the gut (24). b) The release of such transmitters in response to a nerve impulse from the control centre (4) leads to smooth muscle contraction (25).
- Fig. 4:: Panel A and B depict a top-down view of a preferred embodiment of a biological switching circuit (1), comprising a channel system for media flow (2), an inflow-port (17), a pump (15), three valves (16), a waste collector (18), cultures space for the control centre (4), culture spaces for the sender (3) and the responder (5), wherein the culture space of the sender (3) and the responder (5) are connected or the same. The outgrowth of afferent (6) and efferent (7) transmission lines is supported by a channel circuit combining the control centre (4) with a combined sender and responder culture space (3, 5). Panel A depicts a biological switching circuit which comprises a separate channel system for the afferent and efferent signal transmission lines and for the media flow circulation whilst panel B depicts a biological switching circuit wherein the afferent and efferent transmission lines are combined with the media flow circulation. Both systems support the establishment of a biological switching circuit for modelling gut contraction in response to extension. The arrangement of the pump and valves support continuous circulation of culture medium with an option to intermittently or permanently refresh nutrients and remove waste.
- Fig. 5:: Panel A depicts a top-down view of a preferred embodiment of a biological switching circuit (1), comprising a channel system for media flow (2), an inflow-port (17), a pump (15), three valves (16), two waste collectors (18), cultures space for the control centre (4), culture spaces for the sender (3) and the responder (5). The outgrowth of afferent (6) and efferent (7) transmission lines is supported by channels connecting the control centre (4) with the individual culture spaces of a sender (3) and responder (5). Panel A depicts a biological switching circuit comprising a separate channel system for the transmission lines and for the media flow circulation whilst panel B depicts a system where the transmission lines are combined with the media flow channels. Both systems support the establishment of a biological switching circuit for modelling the cognition of impulses to our sense systems on sender (3) site for example by light or heat sensing and modelling the reaction to these impulses on the responder (5) site by contraction / relaxation of muscle cells, production of secrete by gland cells.

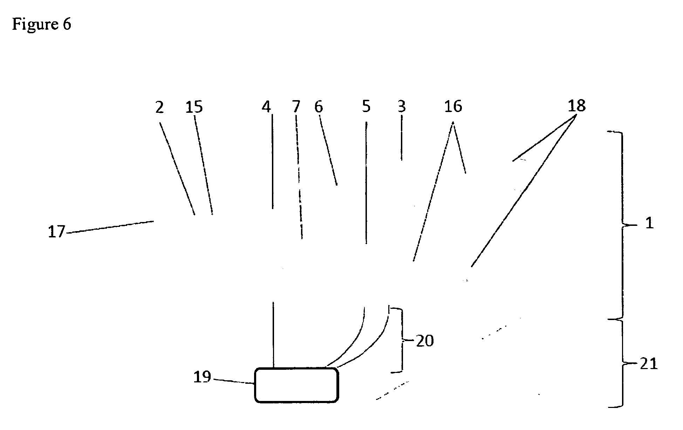

- Fig. 6:: Depicts a three-dimensional view of the biological switching circuit (1) depicted in figure 5a comprising optical sensors (20), controlling the tissue culture spaces for the sender (3) and the control centre (4) and the responder (5). A receiver (19) is completing this monitoring device (21).

### List of reference numbers used

- (1): biological switching circuit
- (2): media flow
- (3): sender
- (4): control centre
- (5): responder
- (6): afferent transmission line
- (7): efferent transmission line
- (8): afferent neuronal cell
- (9): efferent neuronal cell
- (10): glia cell
- (11): axon
- (12): nerve
- (13): coating / connective tissue
- (14): matrix
- (15): pumping device
- (16): valve
- (17): fluid reservoir / inflow-port
- (18): waste collector
- (19): receiver
- (20): sensors
- (21): device for monitoring
- (22): sender tissue before stimulation
- (23): sender tissue after stimulation
- (24): responder tissue before signal
- (25): responder tissue after signal

## Claims

1. A method for assembly of an *in vitro* biological switching circuit (1), comprising the step of seeding a control centre (4), comprising one or more cells capable of forming an afferent transmission line (6), one or more cells capable of forming an efferent transmission line (7) and one or more connective tissue (13) cells, wherein the afferent transmission line (6) formed is capable of connecting at least one sender (3) to the control centre (4) in a way allowing transmission of a signal to the control centre (4) and the efferent transmission line (7) formed is capable of connecting at least one responder (5) to the control centre (4) in a way allowing transmission of a signal to said at least one responder (5).

2. The method of claim 1, further comprising the step of providing at least one sender (3) and/or at least one responder (5).

3. The method of claim 1 or 2, wherein said sender (3) is a cell, a group of cells, or a tissue which is capable of producing a signal in response to a stimulus preferably selected from the group consisting of tension, pressure, changes in volume, shear stress, nutrients, osmolarity, pH, temperature, CO₂, O₂, biochemical changes, light, odor, acoustic noise, taste, colour, and wavelength.

4. The method of any of claims 1 to 3, wherein the one or more cells capable of forming an afferent signal transmission line is/are selected from the group of
a. afferent neuronal cells (8),
b. afferent neuronal cells (8) and glia cells (10)
c. axons (11) of afferent neuronal cells (8) coated with glia cells (10), and/or
d. bundles of axons (11) of afferent neuronal cells (8) with or without glia cell (10) coating combined into nerves (12).

5. The method of any of claims 1 or 4, wherein the at least one efferent signal transmission line is/are
a. efferent neuronal cells (9),
b. efferent neuronal cells (9) and glia cells (10).
c. axons (11) of efferent neuronal cells (9) coated with glia cells (10), and/or
d. bundles of axons (11) of efferent neuronal cells (9) with or without glia cell (10) coating combined into nerves (12).

6. The method of any of claims 1 to 5, wherein the signal transmission lines are nerve (12) fibres.

7. The method of any of claims 1 to 6, wherein the cells forming the control centre (4) comprise or consist of at least one afferent neuronal cell (8), one efferent neuronal cell (9), and at least one connective tissue (13) cell, preferably at least one glia cell (10), arranged in a common structure.

8. The method of any of claims 1 to 7, wherein said responder (5) is a cell, a group of cells or a tissue which is capable of responding to the neuronal signal of the control centre (4), preferably selected from the group consisting of muscle cells, adenocytes, mucosa cells, neuronal cells, a vascular system, nervous system and/or a lymphatic system.

9. The method of any of claims 1 to 8, wherein the one or more cells capable of forming an afferent transmission line (6), one or more cells capable of forming an efferent transmission line (7) and one or more connective tissue (13) cells and optionally the cell, group of cells or tissues forming the sender and/or responder are seeded within a three-dimensional structured matrix (14) and/or on a structured surface.

10. The method of claim 9, wherein the three-dimensional structured matrix (14) and/or structured surface provides an interface for connecting the biological switching unit to at least one technical switching circuit, preferably an electric circuit.

11. The method of any of claims 1 to 10, comprising at least one of the steps of:
a. co-culturing extracted neuronal cells with glia cells (10) supported by a defined assembly architecture,
b. promoting the signal transmission line growth to the sender (3) and the responder (5) cells/tissues,
c. coupling into the sender (3) and/or responder (5),
d. inducing an impulse of the sender (3) and/or
e. measurement of the response at the receiver (19).

12. An in vitro biological switching circuit (1) producible by the method of steps 1 to 11.

13. A kit comprising the biological switching unit comprising the biological switching circuit (1) according to claim 12 and further comprising devices for monitoring (21) the activity of the control centre (4), the sender (3) and/or the responder (5).

14. Use of the biological switching circuit (1) according to claim 12 or the biological switching unit according to claim 13:
a. for research and drug development on neuronal diseases,
b. for safety and efficacy testing of substances,
c. as an implant and
d. as a transplant.

15. Use of the biological switching circuit (1) according to claim 12 or the biological switching unit according to claim 13 as a substitute of technical systems comprising technical switching circuits.
